(19) Europäisches Patentamt
European Patent Office
Office européen des brevets

(11) **EP 3 725 217 A1**

(12) **EUROPEAN PATENT APPLICATION**
published in accordance with Art. 153(4) EPC

(43) Date of publication:
**21.10.2020 Bulletin 2020/43**

(51) Int Cl.:
*A61B 5/024* (2006.01)    *A61B 5/00* (2006.01)
*G06K 9/00* (2006.01)    *G06K 9/46* (2006.01)
*G06N 20/00* (2019.01)

(21) Application number: **19764567.4**

(22) Date of filing: **06.03.2019**

(86) International application number:
**PCT/KR2019/002589**

(87) International publication number:
**WO 2019/172642 (12.09.2019 Gazette 2019/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.03.2018 KR 20180027057**

(71) Applicant: **Samsung Electronics Co., Ltd.**
**Gyeonggi-do 16677 (KR)**

(72) Inventors:
• **KIM, Gyehyun**
**Suwon-si Gyeonggi-do 16677 (KR)**
• **KIM, Joonho**
**Suwon-si Gyeonggi-do 16677 (KR)**
• **KIM, Hyungsoon**
**Suwon-si Gyeonggi-do 16677 (KR)**
• **LEE, Taehan**
**Suwon-si Gyeonggi-do 16677 (KR)**
• **HAN, Jonghee**
**Suwon-si Gyeonggi-do 16677 (KR)**
• **PARK, Sangbae**
**Suwon-si Gyeonggi-do 16677 (KR)**
• **BAEK, Hyunjae**
**Suwon-si Gyeonggi-do 16677 (KR)**

(74) Representative: **HGF**
**1 City Walk**
**Leeds LS11 9DX (GB)**

(54) **ELECTRONIC DEVICE AND METHOD FOR MEASURING HEART RATE**

(57) An electronic device and a method for measuring heart rate are disclosed. A heart rate measuring method of an electronic device, according to the present invention, comprises the steps of: capturing an image including a user's face; grouping the user's face, included in the image, into a plurality of regions including a plurality of pixels of similar colors; acquiring an information on a user's heart rate by inputting information on the plurality of grouped regions to an artificial intelligence learning model; and outputting the acquired information on heart rate. Therefore, the electronic device can measure the user's heart rate more accurately through the captured image.

## FIG. 10

EP 3 725 217 A1

**Description**

[Technical Field]

This disclosure relates to an electronic device for measuring a heart rate and a method for measuring thereof and, more particularly, to an electronic device for measuring a heart rate of a user using a captured image of a face and a method for measuring thereof.

[Background Art]

In a general heart rate measurement method, a sensor is attached to a body portion such as a finger of a user, and the heart rate of the user is measured by using sensing information sensed using an attached sensor.
With the development of an electronic technology, a camera-based non-contact heart rate measurement method for measuring heart rate of a user through an image captured by a camera without attaching a separate sensor to a body portion of a user has been developed.
The camera-based non-contact heart rate measurement method is a method for capturing an image including a face of a user and measuring the heart rate of a user through a color change of the facial skin of a user included in the captured image.
When the user's heart rate is measured from an image of a face captured in event situations such as when the user's face is captured in a state that the facial color is dark or bright due to a surrounding environment (e.g., indoor illumination), or the face of the user is captured when the user's skin is temporarily changed by a movement of the user, the above heart rate measurement method may have a problem in that incorrect heart rate is measured.

[Disclosure]

[Technical Problem]

The objective of the disclosure is to measure a heart rate of a user accurately through an image captured by an electronic device.

[Technical Solution]

According to an embodiment, a method for measuring a heart rate of an electronic device includes capturing an image including a user's face, grouping the user's face, included in the image, into a plurality of regions including a plurality of pixels of similar colors, inputting information on the plurality of grouped regions to an artificial intelligence learning model so as to acquire information on a user's heart rate, and outputting the acquired information on heart rate.
The grouping may include grouping the user's face into a plurality of regions based on color information and position information of the plurality of pixels constituting the user's face, acquiring color values corresponding to each of the plurality of grouped regions, grouping a plurality of regions within a predetermined color range into a same group based on color values corresponding to each of the plurality of acquired regions, and acquiring a pulse signal for a plurality of regions that are grouped into the same group using color values of each of the plurality of regions grouped into the same group.
The acquiring may include acquiring information on a heart rate of the user by inputting the pulse signal for the plurality of regions grouped into the same group to the artificial intelligence learning model.
The artificial intelligence learning model may include a frequencies decompose layer configured to acquire periodic attribute information periodically iterative from the input pulse signal and a complex number layer configured to convert periodic attribute information acquired through the frequencies decompose layer into a value recognizable by the artificial intelligence learning model.
The method may further include acquiring the face region of the user in the captured image, and the acquiring may include acquiring the face region of the user in the captured image using a support vector machine (SVM) algorithm; and
removing eyes, mouth, and neck portions from the acquired face region of the user.
The grouping may include grouping an image of the remaining region in which the regions of the eyes, mouth, and neck portions are removed into a plurality of regions including a plurality of pixels of similar colors.
The removing may include further removing a region of a forehead portion from the user's face region, and the grouping may include grouping the image of a remaining region in which the regions of the eyes, mouth, and forehead portions are removed into a plurality of regions including a plurality of pixels of similar colors.
The grouping may include grouping an image of some regions among the remaining regions in which the eyes, mouth, and forehead portions are removed into a plurality of regions including a plurality of pixels of similar colors, and the some regions may include a region in which a region of the mouth portion is removed.

According to a still another embodiment, an electronic device includes a capturer, an outputter configured to output information on a heart rate; and a processor configured to group a user's face, included in an image captured by the capturer, into a plurality of regions including a plurality of pixels of similar colors, input information on the plurality of grouped regions to an artificial intelligence learning model so as to acquire information on the user's heart rate, and control the outputter to output the acquired information on heart rate.

The processor may group the user's face into a plurality of regions based on color information and position information of the plurality of pixels constituting the user's face and acquire color values corresponding to each of the plurality of grouped regions, and group a plurality of regions within a predetermined color range into a same group based on color values corresponding to each of the plurality of acquired regions and then acquire a pulse signal for a plurality of regions that are grouped into the same group using color values of each of the plurality of regions grouped into the same group.

The processor may acquire information on a heart rate of the user by inputting a pulse signal for the plurality of regions grouped to the same group to the artificial intelligence learning model.

The artificial intelligence learning model may include a frequencies decompose layer configured to acquire periodic attribute information periodically iterative from the input pulse signal and a complex number layer configured to convert periodic attribute information acquired through the frequencies decompose layer into a value recognizable by the artificial intelligence learning model.

The processor may acquire the face region of the user in the captured image using a support vector machine (SVM) algorithm and remove eyes, mouth, and neck portions from the acquired face region of the user.

The processor may group an image of the remaining region in which the regions of the eyes, mouth, and neck portions are removed into a plurality of regions including a plurality of pixels of similar colors.

The processor may further remove a region of a forehead portion from the user's face region, and group the image of a remaining region in which the regions of the eyes, mouth, and forehead portions are removed into a plurality of regions including a plurality of pixels of similar colors.

The processor may group the image of a remaining region in which the regions of the eyes, mouth, and forehead portions are removed into a plurality of regions including a plurality of pixels of similar colors, and the some region may include a region in which the region of the mouth portion is removed.

[Effect of Invention]

According to an embodiment, an electronic device may measure a user's heart rate more accurately through a captured image by grouping the user's face included in the captured image into regions by colors, and using data based on the color values of the grouped regions as an input value of an artificial intelligence (AI) model.

[Description of Drawings]

FIG. 1 is an example diagram illustrating measuring a user's heart rate by an electronic device according to an embodiment;
FIG. 2 is a block diagram illustrating an electronic device providing information on a heart rate of a user according to an embodiment;
FIG. 3 is a detailed block diagram of an electronic device providing information on a heart rate of a user according to an embodiment;
FIG. 4 is an example diagram illustrating an artificial intelligence learning model according to an embodiment;
FIG. 5 is a first example diagram of acquiring a face region of a user from a captured image by a processor according to an embodiment;
FIG. 6 is a second example diagram illustrating acquiring a user's face region of a user from a captured image by a processor according to still another embodiment;
FIG. 7 is a detailed block diagram of a processor of an electronic device for updating and using an artificial intelligence learning model according to an embodiment;
FIG. 8 is a detailed block diagram of a learning unit and an acquisition unit according to an embodiment;
FIG. 9 is an example diagram of learning and determining data by an electronic device and an external server in association with each other according to an embodiment;
FIG. 10 is a flowchart of a method for providing information on the user's heart rate by an electronic device according to an embodiment; and

FIG. 11 is a flowchart of a method for grouping a user's face region into a plurality of regions including a plurality of pixels of similar colors according to an embodiment.

[Detailed Description of Exemplary Embodiments]

Hereinafter, various example embodiments of the disclosure will be described with reference to the accompanying drawings. However, it is to be understood that the disclosure is not limited to specific embodiments, but includes various modifications, equivalents, and/or alternatives according to embodiments of the disclosure. Throughout the accompanying drawings, similar components will be denoted by similar reference numerals.

In this disclosure, the expressions "have," "may have," "including," or "may include" may be used to denote the presence of a feature (e.g., a component, such as a numerical value, a function, an operation, a part, or the like), and does not exclude the presence of additional features.

In this disclosure, the expressions "A or B," "at least one of A and / or B," or "one or more of A and / or B," and the like include all possible combinations of the listed items. For example, "A or B," "at least one of A and B," or "at least one of A or B" includes (1) at least one A, (2) at least one B, (3) at least one A and at least one B all together.

In addition, expressions "first", "second", or the like, used in the disclosure may indicate various components regardless of a sequence and/or importance of the components, may be used in order to distinguish one component from the other components, and do not limit the corresponding components.

It is to be understood that an element (e.g., a first element) is "operatively or communicatively coupled with / to" another element (e.g., a second element) is that any such element may be directly connected to the other element or may be connected via another element (e.g., a third element). On the other hand, when an element (e.g., a first element) is "directly connected" or "directly accessed" to another element (e.g., a second element), it can be understood that there is no other element (e.g., a third element) between the other elements.

Herein, the expression "configured to" can be used interchangeably with, for example, "suitable for," "having the capacity to," "designed to," "adapted to," "made to," or "capable of." The expression "configured to" does not necessarily refer to "specifically designed to" in a hardware sense. Instead, under some circumstances, "a device configured to" may indicate that such a device can perform an action along with another device or part. For example, the expression "a processor configured to perform A, B, and C" may indicate an exclusive processor (e.g., an embedded processor) to perform the corresponding action, or a generic-purpose processor (e.g., a central processor (CPU) or application processor (AP)) that can perform the corresponding actions by executing one or more software programs stored in the memory device.

The electronic device according to various example embodiments may include at least one of, for example, and without limitation, smartphones, tablet personal computer (PC)s, mobile phones, electronic book readers, desktop PCs, laptop PCs, netbook computers, workstations, servers, a personal digital assistant (PDA), a portable multimedia player (PMP), a moving picture experts group phase 1 or phase 2 (MPEG-1 or MPEG-2) audio layer 3 (MP3) player, a medical device, a camera, a wearable device, or the like. The wearable device may include at least one of the accessory type (e.g., a watch, a ring, a bracelet, a wrinkle bracelet, a necklace, a pair of glasses, a contact lens or a head-mounted-device (HMD)), a fabric or a garment-embedded type (e.g., an electronic clothing), a body-attached type (e.g., a skin pad or a tattoo), a bio-implantable circuit, and the like. In some embodiments of the disclosure, the electronic device may include at least one of, for example, and without limitation, a television, a digital video disc (DVD) player, audio, refrigerator, air-conditioner, cleaner, ovens, microwaves, washing machines, air purifiers, set-top boxes, home automation control panels, security control panels, media box (e.g., Samsung HomeSync™, Apple TV™, or Google TV™), game consoles (e.g., Xbox™, PlayStation™), electronic dictionary, electronic key, camcorder, an electronic frame, or the like.

In another example embodiment, the electronic device may include at least one of, for example, and without limitation, a variety of medical devices (e.g., various portable medical measurement devices such as a blood glucose meter, a heart rate meter, a blood pressure meter, or a temperature measuring device), magnetic resonance angiography (MRA), magnetic resonance imaging (MRI), computed tomography (CT), capturing device, or ultrasonic wave device, and the like), navigation system, global navigation satellite system (GNSS), event data recorder (EDR), flight data recorder (FDR), automotive infotainment devices, marine electronic equipment (e.g., marine navigation devices, gyro compasses, and the like), avionics, security devices, car head units, industrial or domestic robots, drones, automatic teller's machine (ATM), points of sales of stores (POS), Internet of Things (IoT) devices (e.g., light bulbs, various sensors, sprinkler devices, fire alarms, thermostats, street lights, toasters, exercise equipment, hot water tanks, heater, boiler, and the like), or the like.

In this disclosure, a term user may refer to a person using an electronic device or an apparatus (for example: artificial intelligence (AI) electronic device) that uses an electronic device.

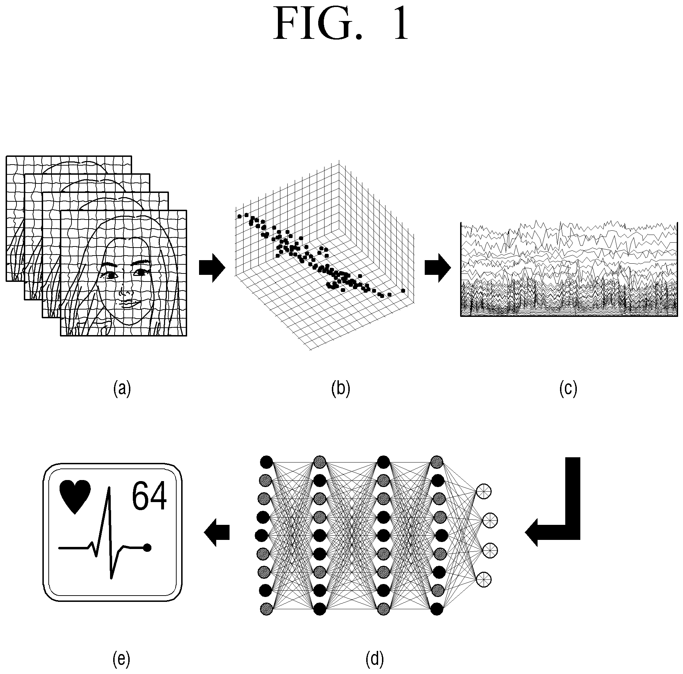

FIG. 1 is an example diagram illustrating measuring a user's heart rate by an electronic device according to an embodiment.

An electronic device 100 may be a device which captures an image and measures a user's heart rate based on an image of a user's face included in the captured image.

The electronic device 100 may be a device such as a smartphone, a tablet personal computer (PC), a smart television (TV), a smart watch, or the like, or a smart medical device capable of measuring the heart rate.

As illustrated in FIG. 1A, if an image is captured, the electronic device 100 may group the user's face, included in the captured image, into a plurality of regions including a plurality of pixels of similar colors.

According to an embodiment, when a user's face region is acquired in an image frame constituting a captured image, the electronic device 100 may group the user's face into a plurality of regions based on color information and location information of a plurality of pixels constituting the acquired user face region.

The electronic device 100 may group pixels having the same color among adjacent pixels into one group based on color information and location information of a plurality of pixels constituting a face region of a user acquired from the captured image.

However, the embodiment is not limited thereto, and the electronic device 100 may group pixels having colors included within a predetermined color range among adjacent pixels into one group based on color information and location information of a plurality of pixels constituting a face region of a user.

The electronic device 100 acquires a color value corresponding to each of the plurality of grouped regions. The electronic device 100 may acquire a color value corresponding to each of the plurality of regions based on color information of pixels included in each of the plurality of grouped regions.

According to an embodiment, the electronic device 100 may calculate an average value from color information of pixels included in each of the plurality of grouped regions and may acquire the calculated average value as a color value corresponding to each of the plurality of grouped regions.

The electronic device 100 then may group a plurality of regions in a predetermined color range into a same group based on the color value corresponding to each of the plurality of regions.

The electronic device 100 may group a plurality of regions in the predetermined color range into the same group using Gaussian distribution.

As shown in FIG. 1B, the electronic device 100 may group a region similar to the A color, among the plurality of regions, into a first group based on color information and position information for each of a plurality of regions constituting the face of the user, group a region similar to the B color into a second group, and group a region similar to the C color into a third group based on color information and position information for each of a plurality of regions constituting the face of the user.

As illustrated in FIG. 1C, the electronic device 100 may acquire a pulse signal for a plurality of grouped regions based on the grouped color value.

As described above, when a plurality of regions are grouped into first to third groups based on color values for each of the grouped regions, the electronic device 100 may acquire a first pulse signal based on a color value for each region included in the first group, acquire a second pulse signal based on a color value for each region included in the second group, and acquire a third pulse signal based on a color value for each region included in the third group.

As illustrated in FIG. 1D, the electronic device 100 may acquire information on the heart rate of a user by inputting a pulse signal for a plurality of grouped regions into an artificial intelligence learning model. The electronic device 100 may output the acquired information on the heart rate of the user as illustrated in FIG. 1E.

Each configuration of the electronic device 100 which provides information on the heart rate of the user by analyzing the region of the user's face included in the captured image will be described in greater detail.

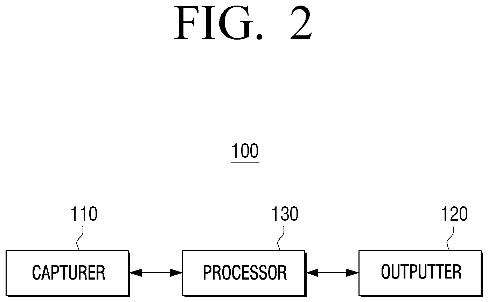

FIG. 2 is a block diagram illustrating an electronic device providing information on a heart rate of a user according to an embodiment.

As illustrated in FIG. 2, the electronic device 100 includes a capturer 110, an outputter 120, and a processor 130.

The capturer 110 captures an image using a camera. The captured image may be a moving image or a still image.

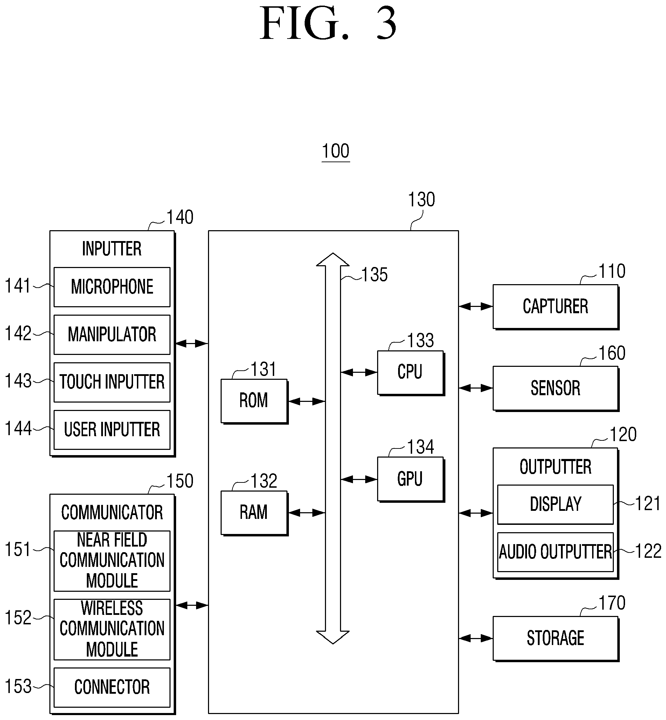

The outputter 120 outputs information on the heart rate of the user acquired based on the face region of the user included in the image captured through the capturer 110. The outputter 120 may include a display 121 and an audio outputter 122 as illustrated in FIG. 3 to be described later.

Therefore, the outputter 120 may output information on the heart rate of the user through at least one of the display 121 and the audio outputter 122.

The processor 130 controls an operation of the configurations of the electronic device 100 in an overall manner.

The processor 130 groups a user's face included in the image captured by the capturer 110 into a plurality of regions including a plurality of pixels of similar colors. The processor 130 then may input information about the plurality of grouped regions into the artificial intelligence learning model to acquire information about the user's heart rate.

The processor 130 then controls the outputter 120 to output information about the acquired heart rate of the user. Accordingly, the outputter 120 may output information about the heart rate of the user through at least one of the display 121 and the audio outputter 122.

The processor 130 may group the user's face into a plurality of regions based on color information and location information of a plurality of pixels constituting the user's face, and then acquire a color value corresponding to each of the plurality of grouped regions.

According to an embodiment, the processor 130 may group pixels having the same color, among adjacent pixels, into one group based on color information and position information of a plurality of pixels constituting the face region of the user.

The embodiment is not limited thereto, and the processor 130 may group pixels having colors included within a predetermined color range among adjacent pixels into one group based on color information and location information of a plurality of pixels constituting a face region of the user.

The processor 130 may calculate an average value from color information of a plurality of pixels included in each of the plurality of grouped regions and may acquire the calculated average value as a color value corresponding to each of the plurality of grouped regions.

The processor 130 may group a plurality of regions in a predetermined color range into the same group based on a color value corresponding to each of the plurality of regions.

The processor 130 may group a plurality of regions in a predetermined color range into the same group using the Gaussian distribution.

The processor 130 may acquire a pulse signal for a plurality of regions grouped into the same group using a color value of a plurality of regions grouped into the same group.

When a pulse signal for a plurality of regions grouped into the same group is acquired, the processor 130 may input a pulse signal for a plurality of regions grouped into the same group to an artificial intelligence learning model to acquire information on the heart rate of the user.

The artificial intelligence learning model may be stored in the storage 170 to be described later, and the artificial intelligence model will be described in greater detail below.

The processor 130 may acquire the user's face region from the image captured through the capturer 110 using the embodiment described below.

When an image is captured through the capturer 110, the processor 130 may acquire a face region of a user within a plurality of image frames constituting an image captured using a support vector machine (SVM) algorithm.

The processor 130 may reduce a noise of a face edge of the user using a confidence map.

According to an embodiment, the processor 130 may reduce noise at the edge of the user's face region using the confidence map based on Equation 1 below.

【Equation 1】

$$inside_{mask} = distance\_transform$$
$$dist_{max} = \log 10.5 - \log 0.5$$
$$inside_{mask} = \log(inside_{mask} + 0.5) - \log 0.5$$

$$inside_{mask} = \begin{cases} dist_{max} & inside_{mask} > dist_{max} \\ inside_{mask} & inside_{mask} \leq dist_{max} \end{cases}$$

$$result_{mask} = \frac{inside_{mask}}{inside_{mask}}$$
$$mask_{W} = \left| \left[ \frac{\sum skin_{map}}{n} \right] - \left[ \frac{\sum result_{mask}}{n} \right] \right|$$
$$mask_{W\_rate} = \begin{cases} 0.5, & mask_{W} > 0.4 \\ 0.05, & mask_{W} \leq 0.4 \end{cases}$$
$$confidence_{map} = [\sum skin] \times (1 - mask_{W\_rate}) + (result_{mask} \times mask_{W\_rate})$$

When the face region of the user is acquired from the captured image through the above-described embodiment, the processor 130 may remove a partial region from the previously acquired face region through a predefined feature point algorithm, and may group the remaining region into a plurality of regions including a plurality of pixels of similar color region after the removal.

According to one embodiment, the processor 130 may detect a region of the eye, mouth, neck portions in the face region of the user that has been already acquired using a predefined feature point algorithm, and may remove detected regions of the eye, mouth, and neck portions.

The processor 130 may group a remaining region of the user's face region from which eyes, mouth, and neck portions are removed into a plurality of regions including a plurality of pixels of the similar color according to an embodiment described above.

According to another embodiment, the processor 130 may detect a region of the user's eye, mouth, neck, and forehead portions when the user's face region is acquired, and may remove regions of the detected user's eyes, mouth, neck and forehead portions.

The processor 130 may group the remaining regions in the user's face from which eyes, mouth, neck, and forehead portions are removed into a plurality of regions including a plurality of pixels of the similar color.

According to still another embodiment, when the user's face region is acquired, the processor 130 may detect the regions of the eyes, mouth, neck, and forehead portions of the user, and may remove the detected regions of the eyes, mouth, neck, and forehead portions.

The processor 130 may group the region of the user's face from which eyes, mouth, neck, and forehead portions are removed, in an image of some of a region among the remaining regions, into a plurality of regions including a plurality of pixels of the same color.

FIG. 3 is a detailed block diagram of an electronic device providing information on a heart rate of a user according to an embodiment.

As described above, the electronic device 100 may further include an inputter 140, a communicator 150, a sensor 160, and a storage 170, as illustrated in FIG. 3, in addition to the configurations of the capturer 110, the outputter 120, and the processor 130.

The inputter 140 is an input means for receiving various user commands and delivering the commands to the processor 130. The inputter 140 may include a microphone 141, a manipulator 142, a touch inputter 143, and a user inputter 144.

The microphone 141 may receive a voice command of a user and the manipulator 142 may be implemented as a key pad including various function keys, number keys, special keys, character keys, or the like.

When the display 121 is implemented in the form of a touch screen, the touch inputter 143 may be implemented as a touch pad that forms a mutual layer structure with the display 121. In this example, the touch inputter 143 may receive a selection command for various application-related icons displayed through the display 121.

The user inputter 144 may receive an infrared (IR) signal or radio frequency (RF) signal for controlling the operation of the electronic device 100 from at least one peripheral device (not shown) such as a remote controller.

The communicator 150 performs data communication with a peripheral device (not shown) such as a smart TV, a smart phone, a tablet PC, a content server (not shown), and a relay terminal device (not shown) for transmitting and receiving data. When the above-described artificial intelligence model is stored in a separate artificial intelligence server (not shown), the communicator 150 may transmit a pulse signal acquired based on the user's face region included in the captured image to the artificial intelligence server (not shown), and may receive information on the heart rate of the user based on the pulse signal from the artificial intelligence server (not shown).

The communicator 150 may include a connector 153 including at least one of a wireless communication module 152 such as a wireless LAN module, and a near field communication module 151 and a wired communication module such as high-definition multimedia interface (HDMI), universal serial bus (USB), institute of electrical and electronics engineers (IEEE) 1394, or the like.

The near field communication module 151 may include various near-field communication circuitry and may be configured to perform near field communication with a peripheral device located at a near distance from the electronic device 100 wirelessly. The near field communication module 131 may include at least one of a Bluetooth module, infrared data association (IrDA) module, near field communication (NFC) module, WI-FI module, and Zigbee module.

The wireless communication module 152 is a module connected to an external network according to wireless communication protocol such as IEEE for performing communication. The wireless communication module may further include a mobile communication module for connecting to a mobile communication network according to various mobile communication specification for performing communication such as 3rd generation (3GT), 3rd generation partnership project (3GPP), long term evolution (LTE), or the like.

The communicator 150 may be implemented as various near field communication method and may employ other communication technology not mentioned in the disclosure if necessary.

The connector 153 is configured to provide interface with various source devices such as USB 2.0, USB 3.0, HDMI, IEEE 1394, or the like. The connector 153 may receive content data transmitted from an external server (not shown) through a wired cable connected to the connector 153 according to a control command of the processor 130, or transmit prestored content data to an external recordable medium. The connector 153 may receive power from a power source through a wired cable physically connected to the connector 153.

The sensor 160 may include an accelerometer sensor, a magnetic sensor, a gyroscope sensor, or the like, and sense a motion of the electronic device 100 using various sensors.

The accelerometer sensor is a sensor for measuring acceleration or intensity of shock of a moving electronic device 100 and is an essential sensor that is used for various transportation means such as a vehicle, a train, an airplane, or the like, and a control system such as a robot as well as the electronic devices such as a smartphone and a tablet PC.

The magnetic sensor is an electronic compass capable of sensing azimuth using earth's magnetic field, and may be used for position tracking, a three-dimensional (3D) video game, a smartphone, a radio, a global positioning system (GPS), a personal digital assistant (PDA), a navigation device, or the like.

The gyroscope sensor is a sensor for applying rotation to an existing accelerometer to recognize a six-axis direction for recognizing a finer and precise operation.

The storage 170 may store an artificial intelligence learning model to acquire information on a heart rate of the user from the pulse signal acquired from the face region of the user, as described above.

The storage 170 may store an operating program for controlling an operation of the electronic device 100.

If the electronic device 100 is turned on, the operating program may be a program that is read from the storage 170 and compiled to operate each configuration of the electronic device 100. The storage 170 may be implemented as at least one of a read only memory (ROM), a random access memory (RAM), or a memory card (for example, secure digital (SD) card, memory stick) detachable to the electronic device 100, non-volatile memory, volatile memory, hard disk drive (HDD), or solid state drive (SSD).

As described above, the outputter 120 includes the display 121 and the audio outputter 122.

As described above, the display 121 displays information on the user's heart rate acquired through the artificial intelligence learning model. The display 121 may display content or may display an execution screen including an icon for executing each of a plurality of applications stored in the storage 170 to be described later or various user interface (UI) screens for controlling an operation of the electronic device 100.

The display 121 may be implemented as a liquid crystal display (LCD), an organic light emitting display (OLED), or the like.

The display 121 may be implemented as a touch screen making a mutual layer structure with the touch inputter 143 receiving a touch command.

As described above, the audio outputter 122 outputs information on the heart rate of the user acquired through the artificial intelligence learning model in an audio form. The audio outputter 122 may output audio data or various alert sound or voice messages included in the content requested by the user.

The processor 130 as described above may be a processing device that controls overall operation of the electronic device 100 or enables controlling of the overall operation of the electronic device 100.

The processor 130 may include a central processing unit 133, a read-only memory ROM 131, a random access memory (RAM) 132, and a graphics processing unit 134, and the CPU 133, ROM 131, RAM 132, and GPU 134 may be connected to each other through a bus 135.

The CPU 133 accesses the storage 170 and performs booting using an operating system (OS) stored in the storage 170, and performs various operations using various programs, contents data, or the like, stored in the storage 170.

The GPU 134 may generate a display screen including various objects such as icons, images, text, and the like. The GPU 134 may calculate an attribute value such as a coordinate value, a shape, a size, and a color to be displayed by each object according to the layout of the screen based on the received control command, and may generate display screens of various layouts including objects based on the calculated attribute value.

The ROM 131 stores one or more instructions for booting the system and the like. When the turn-on instruction is input and power is supplied, the CPU 133 copies the OS stored in the ROM 131 to the RAM 134 according to the stored one or more instructions in the ROM 131, and executes the OS to boot the system. When the booting is completed, the CPU 133 copies various application programs stored in the memory 170 to the RAM 132, executes the application program copied to the RAM 132, and performs various operations.

The processor 130 may be coupled with each configuration and may be implemented as a single chip system (system-on-a-chip, system on chip, SOC, or SoC).

Hereinafter, an artificial intelligence learning model for providing information on the heart rate of a user from a pulse signal acquired based on color information and location information for each of a plurality of pixels constituting a face region of a user will be described in detail.

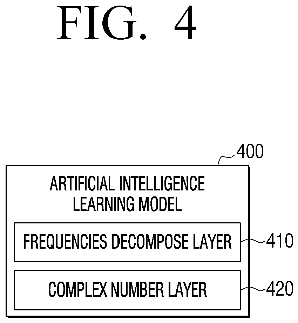

FIG. 4 is an example diagram illustrating an artificial intelligence learning model according to an embodiment.

Referring to FIG. 4, an artificial intelligence learning model 400 includes a frequencies decompose layer 410 and a complex number layer 420.

The frequencies decompose layer 410 acquires periodically iterative periodic attribute information from the input pulse signal.

The complex number layer 420 converts the periodic attribute information input through the frequencies decompose layer 410 as a value recognizable by the artificial intelligence learning model 400.

The frequencies decompose layer 410 receives a pulse signal for a plurality of regions grouped into the same group, as described above. When a pulse signal for a plurality of regions grouped into the same group is input, the frequencies decompose layer 410 acquires periodic attribute information periodically repeated from the pulse signal for each group.

The periodic attribute information may be a complex number value.

When the periodic attribute information, which is a complex number value, is input through the frequencies decompose layer 410, the plurality of layers 420 convert the value to a value recognizable by the artificial intelligence learning model 400. Here, the recognizable value in the artificial intelligence learning model 400 can be a real value.

The artificial intelligence learning model 400 may acquire information on the heart rate of the user using the transformed values in relation to the periodic attribute information acquired from the pulse signal for each group through the complex number layer 420.

Hereinbelow, an operation of acquiring the user's face region from the image captured by the processor 130 will be described in greater detail.

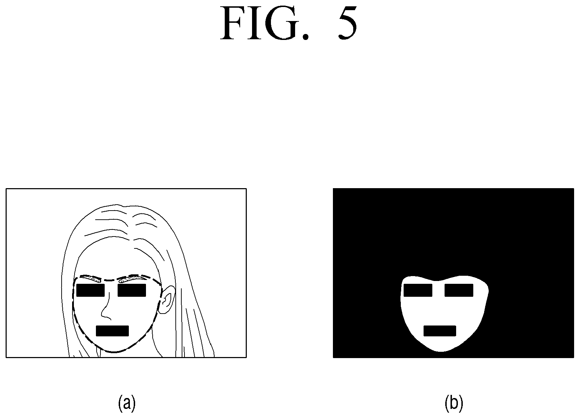

FIG. 5 is a first example diagram of acquiring a face region of a user from a captured image by a processor according to an embodiment.

As illustrated in FIG. 5A, when an image captured through the capturer 110 is input, the processor 130 acquires the user's face region within the image input through the embodiment described above.

The processor 130 may detect a region of the eye, mouth, neck, and forehead within the face region of the user which has already been acquired using the predefined feature point algorithm. The processor 130 then may remove the detected regions of the eye, mouth, neck and forehead within the user's face region.

As illustrated in FIG. 5B, the processor 130 may acquire a face region of the user from which regions of the eye, mouth, neck, and forehead portions have been removed, and may perform grouping into a plurality of regions including a plurality of pixels of the similar color within the face region of the user from which the regions of the eye, mouth, neck, and forehead portions have been removed.

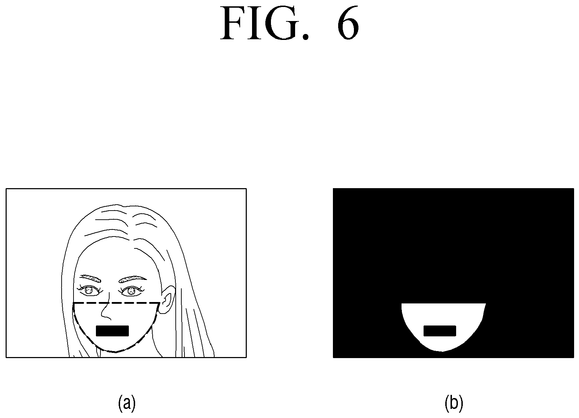

FIG. 6 is a second example diagram illustrating acquiring a user's face region of a user from a captured image by a processor according to still another embodiment.

As illustrated in FIG. 6A, when an image captured through the capturer 110 is input, the processor 130 may acquire the user's face region in the image input through the embodiment described above.

The processor 130 may detect regions of the eye, mouth, neck, and forehead portions in the pre-acquired face region of the user using a predefined feature point algorithm. The processor 130 then removes the detected regions of the eye, mouth, neck, and forehead from the user's face region.

As described above, if the user's face region from which the regions of the eyes, the mouth, the neck, and the forehead portions are removed is acquired, the processor 130 may determine a region to be grouped into a plurality of regions among the face region of the user from which the regions of the eyes, the mouth, the neck, and the forehead portions are removed.

As illustrated in FIG. 6A, the processor 130 determines some regions among the user's face region from which the regions of the eyes, the mouth, the neck, and the forehead portion are removed as a region to be grouped into a plurality of regions. Here, a portion of the region may be a region of a lower portion including a region in which a region of the mouth portion is removed.

Accordingly, as shown in FIG. 6B, the processor 130 may acquire a lower portion of the user's face region from which the regions of the eyes, the mouth, the neck, and the forehead portion have been removed, and may perform grouping into a plurality of regions including a plurality of pixels of similar color within the acquired lower portion region.

Hereinbelow, an operation of updating and using the artificial intelligence learning model by the processor 130 will be described in greater detail.

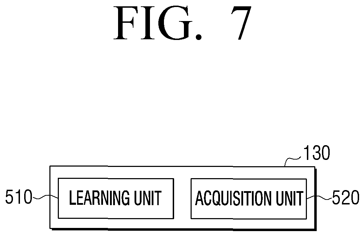

FIG. 7 is a detailed block diagram of a processor of an electronic device for updating and using an artificial intelligence learning model according to an embodiment.

As illustrated in FIG. 7, the processor 130 may include a learning unit 510 and an acquisition unit 520.

The learning unit 510 may generate or train the artificial intelligence learning model for acquiring information on the user's heart rate using the learning data.

The learning data may include at least one of user information, periodic attribute information by pulse signals acquired based on the face image of the user and information on the heart rate by periodic attribute information.

Specifically, the learning unit 510 may generate, train, or update an artificial intelligence learning model for acquiring information on the heart rate of the corresponding user by using the pulse signal acquired based on the color values of the regions grouped in the same group as input data having a similar color distribution in the face region of the user included in the captured image.

The acquisition unit 520 may acquire information on the heart rate of the user by using predetermined data as input data of the pre-learned artificial intelligence learning model.

The acquisition unit 520 may acquire (or recognize, estimate) information about the heart rate of the corresponding user using the pulse signal acquired based on the color values of the regions grouped in the same group as input data having a similar color distribution in the face region of the user included in the captured image.

For example, at least one of the learning unit 510 and the acquisition unit 520 may be implemented as software modules or at least one hardware chip form and mounted in the electronic device 100.

For example, at least one of the learning unit 510 and the acquisition unit 520 may be manufactured in the form of an exclusive-use hardware chip for artificial intelligence (AI), or a conventional general purpose processor (e.g., a CPU or an application processor) or a graphics-only processor (e.g., a GPU) and may be mounted on various electronic devices as described above.

Herein, the exclusive-use hardware chip for artificial intelligence is a dedicated processor for probability calculation, and it has higher parallel processing performance than existing general purpose processor, so it can quickly process computation tasks in artificial intelligence such as machine learning. When the learning unit 510 and the acquisition unit 520 are implemented as a software module (or a program module including an instruction), the software module may be stored in a computer-readable non-transitory computer readable media. In this case, the software module may be provided by an operating system (OS) or by a predetermined application. Alternatively, some of the software modules may be provided by an O/S, and some of the software modules may be provided by a predetermined application.

In this case, the learning unit 510 and the acquisition unit 520 may be mounted on one electronic device 100, or may be mounted on separate electronic devices, respectively. For example, one of the learning unit 510 and the acquisition unit 520 may be implemented in the electronic device 100, and the other one may be implemented in an external server (not shown). In addition, the learning unit 510 and the acquisition unit 520 may provide the model information constructed by the learning unit 510 to the acquisition unit 520 via wired or wireless communication, and provide data which is input to the acquisition unit 520 to the learning unit 510 as additional data.

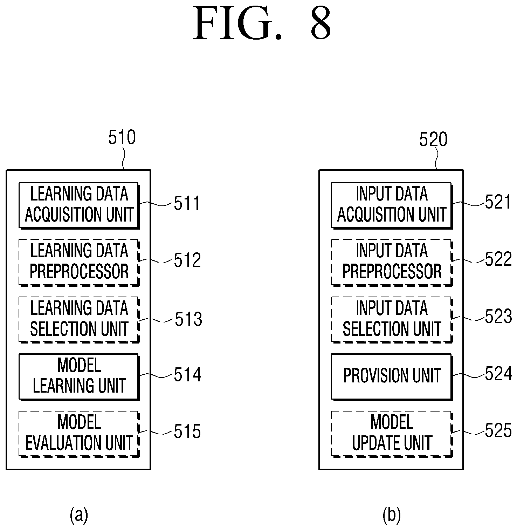

FIG. 8 is a detailed block diagram of a learning unit and an acquisition unit according to an embodiment.

Referring to FIG. 8A, the learning unit 510 according to some embodiments may include a learning data acquisition unit 511 and a model learning unit 514. The learning unit 510 may further selectively implement at least one of a learning data preprocessor 512, a learning data selection unit 513, and a model evaluation unit 515.

The learning data acquisition unit 511 may acquire learning data necessary for the artificial intelligence model. As an embodiment, the learning data acquisition unit 511 may acquire at least one of the periodic attribute information by pulse signals acquired based on the image of the user's face and information on the heart rate by periodic attribute information as learning data.

The learning data may be data collected or tested by the learning unit 510 or the manufacturer of the learning unit 510.

The model learning unit 514 may train, using the learning data, how to acquire periodic attribute information by pulse signals acquired based on the user's face image or information on heart rat4e by periodic attribute information. For example, the model learning unit 514 can train an artificial intelligence model through supervised learning which uses at least a portion of the learning data as a determination criterion.

Alternatively, the model learning unit 514 may learn, for example, by itself using learning data without specific guidance to make the artificial intelligence model learn through unsupervised learning which detects a criterion for determination of a situation.

Also, the model learning unit 514 can train the artificial intelligence model through reinforcement learning using, for example, feedback on whether the result of determination of a situation according to learning is correct.

The model learning unit 514 can also make an artificial intelligence model learn using, for example, a learning algorithm including an error back-propagation method or a gradient descent.

The model learning unit 514 can determine an artificial intelligence model having a great relevance between the input learning data and the basic learning data as an artificial intelligence model to be learned when there are a plurality of artificial intelligence models previously constructed. In this case, the basic learning data may be pre-classified according to the type of data, and the AI model may be pre-constructed for each type of data.

For example, basic learning data may be pre-classified based on various criteria such as a region in which learning data is generated, time at which learning data is generated, the size of learning data, a genre of learning data, a creator of learning data, a type of object within learning data, or the like.

When the artificial intelligence model is learned, the model learning unit 514 can store the learned artificial intelligence model. In this case, the model learning unit 514 can store the learned artificial intelligence model in the storage 170 of the electronic device 100.

Alternatively, the model learning unit 514 may store the learned artificial intelligence model in a memory of a server (for example, an AI server) (not shown) connected to the electronic device 100 via a wired or wireless network.

The learning unit 510 may further implement a learning data preprocessor 512 and a learning data selection unit 513 to improve the response result of the artificial intelligence model or to save resources or time required for generation of the artificial intelligence model.

The learning data pre-processor 512 may pre-process the data associated with the learning to acquire information about periodic attribute information by pulse signals and the user's heart rate based on the periodic attribute information.

The learning data pre-processor 512 may process the acquired data to a predetermined format so that the model learning unit 514 can use data related to learning to acquire information on the heart rate of the user based on the periodic attribute information and the periodic attribute information for each pulse signal.

The learning data selection unit 513 can select data required for learning from the data acquired by the learning data acquisition unit 511 or the data preprocessed by the learning data preprocessor 512. The selected learning data may be provided to the model learning unit 514. The learning data selection unit 513 can select learning data necessary for learning from the acquired or preprocessed data in accordance with a predetermined selection criterion. The learning data selection unit 513 may also select learning data according to a predetermined selection criterion by learning by the model learning unit 514.

The learning unit 510 may further implement the model evaluation unit 515 to improve a response result of the artificial intelligence model.

The model evaluation unit 515 may input evaluation data to the artificial intelligence model, and if the response result which is output from the evaluation result does not satisfy a predetermined criterion, the model evaluation unit may make the model learning unit 514 learn again. In this example, the evaluation data may be predefined data to evaluate the AI learning model.

For example, the model evaluation unit 515 may evaluate, among the recognition results of the learned artificial intelligence learning model for the evaluation data, that the recognition result does not satisfy a predetermined criterion when the number or ratio of the incorrect evaluation data exceeds a preset threshold.

When there are a plurality of learned artificial intelligence learning models, the model evaluation unit 515 can evaluate whether a predetermined criterion is satisfied with respect to each learned artificial intelligence learning model, and determine an artificial intelligence learning model satisfying a predetermined criterion as a final artificial intelligence learning model. In this example, when there are a plurality of artificial intelligence learning models satisfying a predetermined criterion, the model evaluation unit 515 can determine any one or a predetermined number of models preset in the order of high evaluation scores as the final artificial intelligence learning model.

Referring to FIG. 8B, the acquisition unit 520 according to some embodiments may include an input data acquisition unit 521 and a provision unit 524.

In addition, the acquisition unit 520 may further implement at least one of an input data preprocessor 522, an input data selection unit 523, and a model update unit 525 in a selective manner.

The input data acquisition unit 521 may acquire the periodic attribution information by pulse signals acquired based on the image of the user's face and acquire data necessary for acquiring information on the user's heart rate based on the acquired periodic attribute information. The provision unit 524 applies the data acquired by the input data acquisition unit 521 to the AI model to acquire periodic attribute information by pulse signals acquired based on the image of the user's face and may acquire information on the heart rate of the user based on the acquired periodic attribution information.

The provision unit 524 may apply the data selected by the input data preprocessor 522 or the input data selection unit 523 to the artificial intelligence learning model to acquire a recognition result. The recognition result can be determined by an artificial intelligence learning model.

As an embodiment, the provision unit 524 may acquire (estimate) the periodic attribute information from the pulse signal acquired from the input data acquisition unit 521.

As another example, the provision unit 524 may acquire (or estimate) information on the heart rate of the user based on the periodic attribute information acquired from the pulse signal acquired by the input data acquisition unit 521.

The acquisition unit 520 may further include the input data preprocessor 522 and the input data selection unit 523 in order to improve a recognition result of the AI model or save resources or time to provide the recognition result.

The input data pre-processor 522 may pre-process the acquired data so that data acquired for input to the artificial intelligence learning model can be used. The input data preprocessor 522 can process the data in a predefined format so that the provision unit 524 can use data to acquire information about the user's heart rate based on periodic attribute information and periodic attribute information acquired from the pulse signal.

The input data selection unit 523 can select data required for determining a situation from the data acquired by the input data acquisition unit 521 or the data preprocessed by the input data preprocessor 522. The selected data may be provided to the response result provision unit 524. The input data selection unit 523 can select some or all of the acquired or preprocessed data according to a predetermined selection criterion for determining a situation. The input data selection unit 523 can also select data according to a predetermined selection criterion by learning by the model learning unit 524.

The model update unit 525 can control the updating of the artificial intelligence model based on the evaluation of the response result provided by the provision unit 524. For example, the model update unit 525 may provide the response result provided by the provision unit 524 to the model learning unit 524 so that the model learning unit 524 can ask for further learning or updating the AI model.

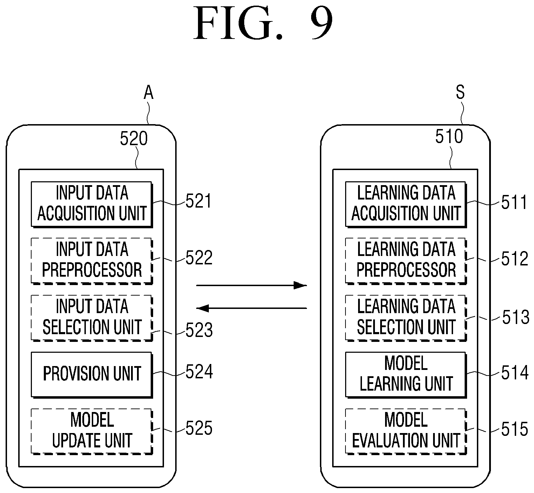

FIG. 9 is an example diagram of learning and determining data by an electronic device and an external server in association with each other according to an embodiment.

As shown in FIG. 9, an external server S may acquire the periodic attribute information from the acquired pulse signal based on the color information and the location information of the user's face region included in the captured image, and may learn the criteria for acquiring information about the heart rate of the user based on the acquired periodic attribute information.

The electronic device (A) may acquire the periodic attribute information from the pulse signal acquired based on the color information and the location information of the face region of the user by using artificial intelligence learning models generated based on the learning result by the server (S), and may acquire information on the heart rate of the user based on the acquired periodic attribute information.

The model learning unit 514 of the server S may perform a function of the learning unit 510 illustrated in FIG. 7. The model learning unit 514 of the server S may learn the determination criteria (or recognition criteria) for the artificial intelligence learning model.

The provision unit 514 of the electronic device A may apply the data selected by the input data selection unit 513 to the artificial intelligence learning model generated by the server S to acquire periodic attribute information from the pulse signal acquired based on the color information and the location information of the face region of the user, and acquire information on the heart rate of the user based on the acquired periodic attribute information.

Alternatively, the provision unit 514 of the electronic device A may receive the artificial intelligence learning model generated by the server S from the server S, acquire periodic attribute information from the pulse signal acquired based on the color information and the location information of the user's face region using the received artificial intelligence learning model, and acquire information about the heart rate of the user based on the acquired periodic attribute information.

It has been described an operation of inputting data acquired from a face region of a user included in an image captured by the electronic device 100 to an artificial intelligence learning model in detail.

Hereinafter, a method for providing information on the heart rate of a user by inputting data acquired from a face region of a user included in an image captured by the electronic device 100 into an artificial intelligence learning model will be described in detail.

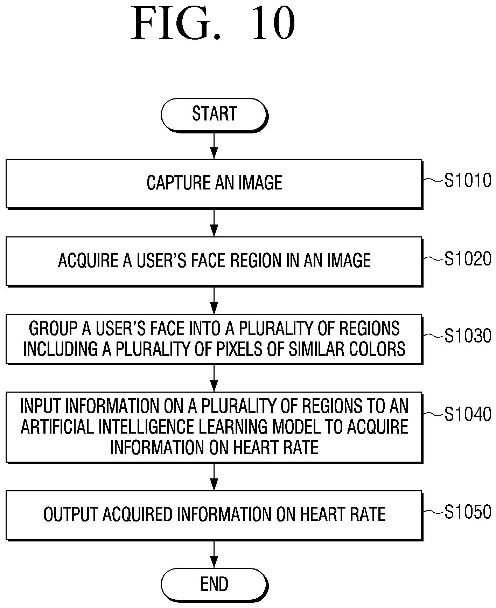

FIG. 10 is a flowchart of a method for providing information on the user's heart rate by an electronic device according to an embodiment.

As illustrated in FIG. 10, the electronic device 100 may capture an image including the user's face and acquire the face region of the user in the captured image in operation S1010.

The electronic device 100 may group the acquired face region into a plurality of regions including a plurality of pixels of the similar color in operation S1020. The electronic device 100 may then acquire information on a user's heart rate by inputting information on a plurality of grouped regions into an artificial intelligence learning model in operation S1030.

The electronic device 100 outputs acquired information on the user's heart rate.

When an image is captured, the electronic device 100 may acquire the user's face region in the pre-captured image using a support vector machine (SVM) algorithm.

If the face region is acquired, the electronic device 100 may remove the regions of the eyes, mouth, and neck portions from the acquired user's face region and may acquire the user's face region from which the eyes, mouth, and neck portions are deleted.

The electronic device 100 may group the face region of the user from which the eyes, mouth, and neck portions are removed into a plurality of regions including a plurality of pixels of the similar color.

According to an additional aspect, the electronic device 100 removes regions of the eye, mouth, neck and forehead portions in the acquired face region once the user's face region is acquired in the captured image. The electronic device 100 may then group the face region into a plurality of regions that include a plurality of pixels of the similar color within the face region of the user from which the regions of the eye, mouth, neck, and forehead portions have been removed.

The electronic device 100 removes regions of the eye, mouth, neck and forehead parts in the acquired face region once the user's face region is acquired in the captured image. The electronic device 100 then groups the regions of the eye, mouth, neck and forehead portions into a plurality of regions including a plurality of pixels of the similar color within some regions of the user's face region. Here, some regions may include regions where regions of the mouth region are removed.

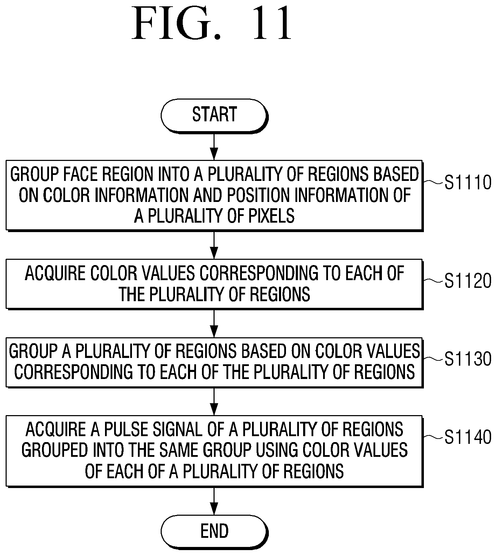

FIG. 11 is a flowchart of a method for grouping a user's face region into a plurality of regions including a plurality of pixels of similar colors according to an embodiment.

As illustrated in FIG. 11, when a face region of a user is acquired from a captured image, the electronic device 100 groups a face region of a user into a plurality of regions based on color information and location information of a plurality of pixels constituting a face region of a user in operation S1110.

Thereafter, the electronic device 100 may acquire a color value corresponding to each of the plurality of grouped regions and may group a plurality of regions within a predetermined color range into the same group based on the color value corresponding to each of the plurality of acquired regions in operations S1120 and S1130.

The electronic device 100 may acquire a pulse signal for a plurality of regions grouped into the same group using a color value of a plurality of regions grouped into the same group in operation S1140.

Through the embodiment, when a pulse signal for the face region of the user is acquired, the electronic device 100 acquires information on the heart rate of the user by inputting the acquired pulse signal to the artificial intelligence learning model.

When a pulse signal is input, the artificial intelligence learning model acquires periodic attribute information periodically repeated from the pulse signal previously input through the frequencies decompose layer. Thereafter, the artificial intelligence learning model converts the periodic attribute information acquired from the frequencies decompose layer into a value recognizable in the artificial intelligence learning model through a plurality of layers.

The periodic attribute information may be a complex number value and a value recognizable in the artificial intelligence learning model may be a real number value.

Accordingly, the artificial intelligence learning model provides information on the heart rate of the user based on the periodic attribute information converted into a value recognizable in the artificial intelligence learning model through the complex number layer. Accordingly, the electronic device 100 may output information provided through the artificial intelligence learning model as information on the heart rate of the user.

In addition, the control method of the electronic device 100 as described above may be implemented as at least one execution program for executing the control method of the image forming apparatus as described above, and the execution program may be stored in a non-transitory computer readable medium.

Non-transitory readable medium means a medium that stores data for a short period of time such as a register, a cache, and a memory, but semi-permanently stores data and is readable by a device. The above programs may be stored in various types of recording medium readable by a terminal, including a random access memory (RAM), a flash memory, a read only memory (ROM), erasable programmable ROM (EPROM), electronically erasable and programmable ROM (EEPROM), a register, a hard disk, a memory card, a universal serial bus (USB) memory, a compact disc read only memory (CD-ROM), or the like.

The preferred embodiments have been described.

Although the examples of the disclosure have been illustrated and described hereinabove, the disclosure is not limited to the abovementioned specific examples, but may be variously modified by those skilled in the art to which the disclosure pertains without departing from the scope and spirit of the disclosure as disclosed in the accompanying claims. These modifications should also be understood to fall within the scope of the disclosure.

**Claims**

1. A method for measuring a heart rate of an electronic device, the method comprising:

   capturing an image including a user's face;
   grouping the user's face, included in the image, into a plurality of regions including a plurality of pixels of similar colors;
   acquiring an information on a user's heart rate by inputting information on the plurality of grouped regions to an artificial intelligence learning model; and
   outputting the acquired information on heart rate.

2. The method of claim 1, wherein the grouping comprises:

   grouping the user's face into a plurality of regions based on color information and position information of the plurality of pixels constituting the user's face;
   acquiring color values corresponding to each of the plurality of grouped regions;
   grouping a plurality of regions within a predetermined color range into a same group based on color values corresponding to each of the plurality of acquired regions; and
   acquiring a pulse signal for a plurality of regions that are grouped into the same group using color values of each of the plurality of regions grouped into the same group.

3. The method of claim 2, wherein the acquiring comprises acquiring information on a heart rate of the user by inputting the pulse signal for the plurality of regions grouped into the same group to the artificial intelligence learning model.

4. The method of claim 3, wherein the artificial intelligence learning model

comprises:

a frequencies decompose layer configured to acquire periodic attribute information periodically iterative from the input pulse signal; and
a complex number layer configured to convert periodic attribute information acquired through the frequencies decompose layer into a value recognizable by the artificial intelligence learning model.

5. The method of claim 1, further comprising:

acquiring the face region of the user in the captured image,
wherein the acquiring comprises:

acquiring the face region of the user in the captured image using a support vector machine (SVM) algorithm; and
removing eyes, mouth, and neck portions from the acquired face region of the user.

6. The method of claim 5, wherein the grouping comprises grouping an image of the remaining region in which the regions of the eyes, mouth, and neck portions are removed into a plurality of regions including a plurality of pixels of similar colors.

7. The method of claim 5, wherein:
the removing comprises further removing a region of a forehead portion from the user's face region, and
the grouping comprises grouping the image of a remaining region in which the regions of the eyes, mouth, and forehead portions are removed into a plurality of regions including a plurality of pixels of similar colors.

8. The method of claim 5, wherein the grouping comprises grouping an image of some regions among the remaining regions in which the eyes, mouth, and forehead portions are removed into a plurality of regions including a plurality of pixels of similar colors, and
wherein the some regions comprise a region in which a region of the mouth portion is removed.

9. An electronic device comprising:

a capturer;
an outputter configured to output information on a heart rate; and
a processor configured to:
group a user's face, included in an image captured by the capturer, into a plurality of regions including a plurality of pixels of similar colors, acquire information on the user's heart rate by inputting an information on the plurality of grouped regions to an artificial intelligence learning model, and control the outputter to output the acquired information on heart rate.

10. The electronic device of claim 9, wherein the processor is further configured to:

group the user's face into a plurality of regions based on color information and position information of the plurality of pixels constituting the user's face and acquire color values corresponding to each of the plurality of grouped regions,
group a plurality of regions within a predetermined color range into a same group based on color values corresponding to each of the plurality of acquired regions and then acquire a pulse signal for a plurality of regions that are grouped into the same group using color values of each of the plurality of regions grouped into the same group.

11. The electronic device of claim 10, wherein the processor is further configured to acquire information on a heart rate of the user by inputting a pulse signal for the plurality of regions grouped to the same group to the artificial intelligence learning model.

12. The electronic device of claim 11, wherein the artificial intelligence learning model comprises:
a frequencies decompose layer configured to acquire periodic attribute information periodically iterative from the input pulse signal; and

a complex number layer configured to convert periodic attribute information acquired through the frequencies decompose layer into a value recognizable by the artificial intelligence learning model.

13. The electronic device of claim 9, wherein the processor is further configured to acquire the face region of the user in the captured image using a support vector machine (SVM) algorithm and remove eyes, mouth, and neck portions from the acquired face region of the user.

14. The electronic device of claim 13, wherein the processor is further configured to group an image of the remaining region in which the regions of the eyes, mouth, and neck portions are removed into a plurality of regions including a plurality of pixels of similar colors.

15. The electronic device of claim 13, wherein the processor is configured to further remove a region of a forehead portion from the user's face region, and group the image of a remaining region in which the regions of the eyes, mouth, and forehead portions are removed into a plurality of regions including a plurality of pixels of similar colors.

# FIG. 1

(a)  (b)  (c)

(e)  (d)

# FIG. 2

100

| 110 | 130 | 120 |
|---|---|---|
| CAPTURER | PROCESSOR | OUTPUTTER |

# FIG. 3

# FIG. 4

# FIG. 5

(a)

(b)

# FIG. 6

(a)

(b)

# FIG. 7

130

510 — LEARNING UNIT | ACQUISITION UNIT — 520

# FIG. 8

510

| | |
|---|---|
| LEARNING DATA ACQUISITION UNIT | 511 |
| LEARNING DATA PREPROCESSOR | 512 |
| LEARNING DATA SELECTION UNIT | 513 |
| MODEL LEARNING UNIT | 514 |
| MODEL EVALUATION UNIT | 515 |

(a)

520

| | |
|---|---|
| INPUT DATA ACQUISITION UNIT | 521 |
| INPUT DATA PREPROCESSOR | 522 |
| INPUT DATA SELECTION UNIT | 523 |
| PROVISION UNIT | 524 |
| MODEL UPDATE UNIT | 525 |

(b)

# FIG. 9

A
520
INPUT DATA ACQUISITION UNIT — 521
INPUT DATA PREPROCESSOR — 522
INPUT DATA SELECTION UNIT — 523
PROVISION UNIT — 524
MODEL UPDATE UNIT — 525

S
510
LEARNING DATA ACQUISITION UNIT — 511
LEARNING DATA PREPROCESSOR — 512
LEARNING DATA SELECTION UNIT — 513
MODEL LEARNING UNIT — 514
MODEL EVALUATION UNIT — 515

# FIG. 10

START

CAPTURE AN IMAGE — S1010

ACQUIRE A USER'S FACE REGION IN AN IMAGE — S1020

GROUP A USER'S FACE INTO A PLURALITY OF REGIONS INCLUDING A PLURALITY OF PIXELS OF SIMILAR COLORS — S1030

INPUT INFORMATION ON A PLURALITY OF REGIONS TO AN ARTIFICIAL INTELLIGENCE LEARNING MODEL TO ACQUIRE INFORMATION ON HEART RATE — S1040

OUTPUT ACQUIRED INFORMATION ON HEART RATE — S1050

END

# FIG. 11

START

GROUP FACE REGION INTO A PLURALITY OF REGIONS BASED ON COLOR INFORMATION AND POSITION INFORMATION OF A PLURALITY OF PIXELS — S1110

ACQUIRE COLOR VALUES CORRESPONDING TO EACH OF THE PLURALITY OF REGIONS — S1120

GROUP A PLURALITY OF REGIONS BASED ON COLOR VALUES CORRESPONDING TO EACH OF THE PLURALITY OF REGIONS — S1130

ACQUIRE A PULSE SIGNAL OF A PLURALITY OF REGIONS GROUPED INTO THE SAME GROUP USING COLOR VALUES OF EACH OF A PLURALITY OF REGIONS — S1140

END

## INTERNATIONAL SEARCH REPORT

| International application No. |
| --- |
| **PCT/KR2019/002589** |

**A. CLASSIFICATION OF SUBJECT MATTER**

*A61B 5/024(2006.01)i, A61B 5/00(2006.01)i, G06K 9/00(2006.01)i, G06K 9/46(2006.01)i, G06N 20/00(2019.01)i*

According to International Patent Classification (IPC) or to both national classification and IPC

**B. FIELDS SEARCHED**

Minimum documentation searched (classification system followed by classification symbols)
A61B 5/024; A61B 5/00; A61B 5/0205; A61B 5/11; A61B 5/1455; G06K 9/46; G06T 7/00; G06K 9/00; G06N 20/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: heart beat, image, image, pixel, grouping, artificial intelligence learning model

**C. DOCUMENTS CONSIDERED TO BE RELEVANT**

| Category* | Citation of document, with indication, where appropriate, of the relevant passages | Relevant to claim No. |
| --- | --- | --- |
| Y | KR 10-2017-0028113 A (XERON HEALTHCARE CORP.) 13 March 2017<br>See claims 1-10; paragraphs [0038]-[0056]. | 1-15 |
| Y | KR 10-2015-0093036 A (ELECTRONICS AND TELECOMMUNICATIONS RESEARCH INSTITUTE) 17 August 2015<br>See claim 1; paragraphs [0035]-[0046]. | 1-15 |
| A | KR 10-2015-0059631 A (SAMSUNG ELECTRONICS CO., LTD.) 01 June 2015<br>See the entire document. | 1-15 |
| A | KR 10-1777472 B1 (SOONCHUNHYANG UNIVERSITY INDUSTRY ACADEMY COOPERATION FOUNDATION) 12 September 2017<br>See the entire document. | 1-15 |
| A | WO 2017-121834 A1 (KONINKLIJKE PHILIPS N.V.) 20 July 2017<br>See the entire document. | 1-15 |
| A | US 9451905 B2 (KONINKLIJKE PHILIPS N.V.) 27 September 2016<br>See the entire document. | 1-15 |

| ☐ Further documents are listed in the continuation of Box C. | ☒ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 10 JUNE 2019 (10.06.2019) | **11 JUNE 2019 (11.06.2019)** |

| Name and mailing address of the ISA/KR | Authorized officer |
| --- | --- |
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

INTERNATIONAL SEARCH REPORT
Information on patent family members

International application No.

**PCT/KR2019/002589**

| Patent document cited in search report | Publication date | Patent family member | Publication date |
|---|---|---|---|
| KR 10-2017-0028113 A | 13/03/2017 | KR 10-1787828 B1 | 19/10/2017 |
| KR 10-2015-0093036 A | 17/08/2015 | None | |
| KR 10-2015-0059631 A | 01/06/2015 | JP 2015-100432 A | 04/06/2015 |
| | | JP 6349075 B2 | 27/06/2018 |
| | | US 2015-0148687 A1 | 28/05/2015 |
| KR 10-1777472 B1 | 12/09/2017 | EP 3318179 A2 | 09/05/2018 |
| | | WO 2017-003049 A2 | 05/01/2017 |
| WO 2017-121834 A1 | 20/07/2017 | CN 108471989 A | 31/08/2018 |
| | | EP 3402402 A1 | 21/11/2018 |
| | | JP 2019-505263 A | 28/02/2019 |
| | | US 2019-0000391 A1 | 03/01/2019 |
| US 9451905 B2 | 27/09/2016 | CN 104010571 A | 27/08/2014 |
| | | CN 104010571 B | 30/01/2018 |
| | | EP 2747649 A1 | 02/07/2014 |
| | | JP 2015-506186 A | 02/03/2015 |
| | | JP 6073919 B2 | 01/02/2017 |
| | | US 2014-0358017 A1 | 04/12/2014 |
| | | WO 2013-093690 A1 | 27/06/2013 |

Form PCT/ISA/210 (patent family annex) (January 2015)